# EUROPEAN PATENT APPLICATION

(11) **EP 4 523 521 A1**
(43) Date of publication of application: **19.03.2025**
(21) Application number: 24198371.7
(22) Date of filing: 04.09.2024
(51) Int. Cl.: A01G 33/00, C12N 1/12

(54) **A PROCESS FOR PREPARING A CELL CULTURE MEDIUM**

(30) Priority: 05.09.2023 IT 202300018219
(71) Applicant: Università Degli Studi di Pavia, 27100 Pavia (PV) (IT)
(72) Inventor: PINNOLA, Alberta, 27010 BORGARELLO (PAVIA) (IT); FORNERIS, Federico, 27100 PAVIA (IT); BARERA, Simone, 13040 PALAZZOLO VERCELLESE (VERCELLI) (IT)
(74) Representative: Delbarba, Andrea

(57) **Abstract**

The present invention relates to a process for preparing a cell culture medium. Furthermore, the invention relates to a culture medium obtained with the process described and a method for promoting or increasing cell growth, preferably of algal cells.

## Description

### FIELD OF THE INVENTION

The present invention relates to a process for preparing a cell culture medium for culturing microalgae using exhausted media recovered from other cell cultures.

### STATE OF THE ART

The biomedical and biopharmaceutical industries intensively use systems based on mixtures of nutrients with which cell culture media are generated. The associated production of waste consisting of exhausted cell culture media is enormous. The disposal of this waste requires decontamination procedures, which are generally carried out using chemical agents such as, for example, hypochlorite and bleach. At present there are no available strategies for reducing or recycling this waste or the content thereof in order to reduce its environmental impact. Moreover, chemical decontamination represents an environmental problem in itself. The possibility of recycling culture media would offer economic and environmental advantages and advantages in terms of efficiency by reducing the use of water and further nutrients. However, this represents a major challenge, yet to be resolved.

Therefore, there is a greatly felt need to obtain a process for recycling and for preparing culture media using media that are exhausted and/or were used in previous cell cultures.

### SUMMARY OF THE INVENTION

A first aspect of the present invention relates to a process for preparing a cell culture medium comprising the steps of:
a) providing an exhausted culture medium;
b) removing cells and/or parts of cells from the exhausted culture medium;
c) sterilising the exhausted medium obtained in step b) to obtain a cell culture medium;
d) optionally mixing the cell culture medium obtained in step c) with at least one nutrient and/or additive;
wherein
sterilisation step c) takes place at a temperature above 100°C for a duration between 5 and 40 minutes and at a pressure between 1 and 3 bar.

Preferably, sterilisation step c) takes place at a temperature between 100 and 150°C for a duration between 10 and 30 minutes and at a pressure between 1.5 and 3 bar.

In one embodiment, sterilisation step c) takes place by filtration with a permeability of less than 0.45 µm, preferably with a permeability of less than 0.2 µm.

Preferably, the exhausted culture medium comprises or consists of a prokaryotic cell culture medium optionally supplemented with at least one antibiotic and/or elements suitable for bacterial cell culture.

Preferably, the exhausted culture medium comprises or consists of a eukaryotic cell culture medium optionally supplemented with at least one antibiotic and/or elements suitable for eukaryotic cell culture.

In one embodiment, step b) is implemented by means of at least one centrifugation and/or filtration process.

A second aspect of the present invention relates to a culture medium obtained/obtainable with the above-described process.

A third aspect of the present invention relates to a method for promoting or increasing the growth of an algae cell culture comprising the steps of:
a) providing an exhausted culture medium;
b) removing cells and/or parts of cells from the exhausted culture medium;
c) sterilising the exhausted medium obtained in step b) to obtain a cell culture medium,
d) optionally mixing the cell culture medium obtained in step c) with at least one nutrient and/or additive;
e) inoculating a plurality of algae cells into the medium obtained in step d), and
f) keeping the plurality of cells in the culture medium for at least 48 hours.

Preferably, the microalgae belong to at least one genus chosen from: Amphora, Anabaena, Anacystis, Apistonema, Arthrospira (Spirulina), Botryococcus, Brachiomonas, Chlamydomonas, Chlorella, Chloroccum, Cruciplacolithus, Cylindrotheca, Coenochloris, Cyanophora, Cyclotella, Dunaliella, Emiliania, Euglena, Extubocellulus, Fragilaria, Galdieria, Goniotrichium, Haematococcus. Halochlorella, Isochyrsis, Leptocylindrus, Micractinium, Melosira, Monodus, Nostoc, Nannochloris, Nannoehloropsis, Navicula, Neospongiococcum, Nitzschia, Odontella, Ochromonas, Ochrosphaera, Pavlova, Parachlorella, Picochlorum, Phaeodactylum, Pleurochyrsis, Porphyridium, Poteriochromonas, Prymnesium. Rhodomonas, Scenedesmus, Skeletonema, Spumella, Stauroneis, Stichococcus, Auxenochlorella, Cheatoceros, Neochloris, Ocromonas, Porphiridium, Synechococcus, Synechocyslis, Tetraselmis, Thraustochytrids, Thalassiosira and species thereof.

Preferably, the microalgae belong to the genus Chlorella, preferably of the species *C*. *vulgaris,* or to the genus Scenedesmus, preferably of the species *S. obliquus,* or to the genus Parachlorella, preferably of the species *P. kessleri,* or to the genus Botryococcus, preferably of the species *B. braunii* and/or to the genus Hematococcus, preferably of the species *H. pluvialis.*

In one embodiment, the method further comprises the steps of:
g) collecting the plurality of cells obtained in step f) preferably by means of centrifugation and/or filtration approaches,
h) drying the plurality of cells obtained from step g) to obtain a dry mass,
i) weighing the dry mass obtained in step h),
j) carrying out a cell culture of microalgae, using at least one culture medium not previously used, and
k) comparing the dry biomass obtained in step i) with the dry biomass obtained in step j) using the following formula:
   weight of dry mass obtained in step i) / weight of dry mass obtained in step j), resulting in a ratio,
wherein the ratio obtained is higher than 2, preferably between 2 and 10; the ratio obtained in step i) is preferably between 2 and 10.

### BRIEF DESCRIPTION OF THE FIGURES

Figure 1 shows the growth of an example strain of microalgae (*C*. *vulgaris*) in different formulations of exhausted culture media in 12-well plates and a comparison with the standard medium (BG11, optionally supplemented with 0.1% acetic acid). A) Growth of the microalgae in plates from day 0 (start of the experiment) to day 7. B) Analysis of microalgal biomass growth (measured as absorption of total chlorophylls drawn from the cell background over time);
Figure 2 shows the growth of microalgae (*C*. *vulgaris*) in different formulations of exhausted culture media in a volume of 30 mL. A) Growth of the microalgae in a flask from day 1 to day 7. B) Analysis of microalgal concentration over time;
Figure 3 shows an example setup of a 400 mL culture in a flask, wherein different formulations were analysed. In this case it may clearly be seen that the dry weight of the microalgal biomass (g/L) in the formulations we tested increased 2 to 10 times compared to standard conditions depending on the days considered and the formulation used;
Figure 4 shows a 30-litre culture of *C*. *vulgaris* microalgae in a photobioreactor.

### DEFINITIONS

In the context of the present invention, the term "exhausted culture medium" is understood to mean a liquid culture medium that was previously used, i.e. previously placed in contact with a plurality of cells and thus normally to be discarded through disposal procedures.

### DETAILED DESCRIPTION OF THE INVENTION

A first aspect of the present invention relates to a process for preparing a cell culture medium. In one embodiment, the process comprises at least a step of recovering an exhausted culture medium. In other words, the process of the present invention allows an exhausted culture medium to be recovered and/or recycled.

In one embodiment, the process comprises the steps of:
a) providing an exhausted culture medium;
b) removing cells and/or parts of cells from the exhausted culture medium;
c) sterilising the exhausted medium obtained in step b) to obtain a cell culture medium, and
d) optionally mixing the cell culture medium obtained in step c) with at least one nutrient and/or additive to obtain a formulation to be subsequently used for microalgae cultures;

In one embodiment, the exhausted culture medium is a medium used by laboratories in cell culture protocols. Preferably, the exhausted culture medium was previously used to culture bacterial cells or to culture eukaryotic cells. In other words, the exhausted culture medium is a culture medium already used for previous cell cultures, preferably for bacterial cell cultures.

In one embodiment, the exhausted culture medium comprises or consists of a bacterial cell culture medium optionally supplemented with at least one antibiotic and/or elements useful for bacterial cell culture. For example, said medium is a Luria Bertani (LB) medium or a medium suitable for cell culture known to the person skilled in the art.

In one embodiment, the exhausted culture medium comprises or consists of a basic medium for eukaryotic cell culture optionally supplemented with at least one antibiotic and/or elements useful for eukaryotic cell culture. For example, said medium is a medium suitable for cell culture known to the person skilled in the art.

Preferably, step b) is implemented by means of at least one centrifugation and/or filtration process.

In one embodiment, the filtration is chosen from: cross-flow filtration, deadend filtration, membrane filtration, vacuum filtration, surface filtration, solid sieves, screen filters, band filters, hollow fibre filters, sheet/plate filters, spiral filters and paper filters. In one embodiment, separation adjuvants can also be used to favour filtration.

In one embodiment, sterilisation step c) takes place at a temperature above 100°C, preferably at a temperature between 100 and 150°C, more preferably at a temperature between 110 and 130°C.

In one embodiment, sterilisation step c) has a duration of between 5 and 40 minutes, preferably between 10 and 30 minutes, more preferably between 15 and 25 minutes.

In one embodiment, sterilisation step c) takes place at a pressure between 1 and 3 bar, preferably between 1.5 and 3 bar, more preferably between 1.5 and 2.5 bar.

Alternatively, sterilisation step c) takes place by filtration with a permeability of less than 0.45 µm, preferably with a permeability of less than 0.2 µm.

In one embodiment, the sterilisation by filtration described in step c) is chosen from: membrane filtration, tangential flow filtration, vacuum filtration, hollow fibre filters, and sheet/plate filters. Preferably, at the end of step c), the exhausted culture medium recovered is kept in a closed container, for example a bottle, at a temperature between 3 and 25°C until the use thereof.

During step d), the recovered exhausted culture medium is preferably mixed with other exhausted culture media in amounts ranging from 0 to 100%.

During step d), the culture medium is preferably mixed with sterile water and/or with standard cell culture medium (BG11) in amounts ranging from 0 to 80%.

The culture medium obtained at the end of step d) with the process of the present invention is used in subsequent cell cultures for the culture of microalgae.

Therefore, at the end of the above-described process, the culture medium obtained is used in subsequent cell cultures, preferably for the culture of algae, even more preferably for the culture of microalgae.

In one embodiment, the microalgae cells can be inoculated into a subsequent culture wherein the rate of growth of the microalgae, calculated as grams of dry weight per litre of medium at the end of the culture itself, is greater compared to a microalgae culture in a medium not obtained with the process of the present invention, i.e. with a culture medium not used in a previous culture.

A second aspect of the present invention relates to a culture medium obtained/obtainable with the process described above in detail. A third aspect of the present invention relates to a method for increasing the growth of an algae cell culture. Said method comprises or consists of the steps of:
a) providing an exhausted culture medium;
b) removing cells and/or parts of cells from the exhausted culture medium;
c) sterilising the exhausted medium obtained in step b) to obtain raw materials to be used for the new cell culture medium;
d) mixing one or more sterilised media obtained in step c), optionally also supplemented with other nutrients and/or additives to obtain new formulations to be subsequently used for culturing microalgae;
e) inoculating a plurality of algae cells into the medium obtained in step d) and
f) keeping the plurality of microalgal cells in the culture medium for at least 48 hours.

In one embodiment, steps a) - e) are implemented as described above in detail for the process.

In one embodiment, the algae are microalgae and preferably belong to at least one genus chosen from: Amphora, Anabaena, Anacystis, Apistonema, Arthrospira (Spirulina), Botryococcus, Brachiomonas. Chlamydomonas, Chlorella, Chloroccum, Cruciplacolithus, Cylindrotheca, Coenochloris, Cyanophora, Cyclotella, Dunaliella, Emiliania, Euglena, Extubocellulus, Fragilaria, Galdieria, Goniotrichium, Haematococcus. Halochlorella, Isochyrsis, Leptocylindrus, Micractinium, Melosira, Monodus, Nostoc, Nannochloris, Nannoehloropsis, Navicula, Neospongiococcum, Nitzschia, Odontella, Ochromonas, Ochrosphaera, Pavlova, Parachlorella, Picochlorum, Phaeodactylum, Pleurochyrsis, Porphyridium, Poteriochromonas, Prymnesium. Rhodomonas, Scenedesmus, Skeletonema, Spumella, Stauroneis, Stichococcus, Auxenochlorella, Cheatoceros, Neochloris, Ocromonas, Porphiridium, Synechococcus, Synechocyslis, Tetraselmis, Thraustochytrids, Thalassiosira and species thereof.

Preferably, the microalgae belong to the genus Chlorella, preferably of the species *C*. *vulgaris,* or to the genus Scenedesmus, preferably of the species *S. obliquus,* or to the genus Parachlorella, preferably of the species *P. kessleri,* or to the genus Botryococcus, preferably of the species *B. braunii* and/or to the genus Hematococcus, preferably of the species *H. pluvialis.*

In one embodiment, the culture medium of step d) comprises at least 20% volume/volume (v/v) of the culture medium obtained in step c) or d), preferably at least 40% v/v or at least 50% v/v or at least 60% v/v or at least 70% v/v or at least 80% v/v or at least 90% v/v or more of the medium obtained in step c) or d). In a preferred embodiment of the invention, the culture medium of step d) comprises at least 80% v/v of the medium obtained in step c).

In one embodiment, in step e) the cells are kept in culture for a time of between 2 and 10 days, preferably between 2 and 7 days.

Preferably, the method further comprises the following steps:
g) collecting the algal biomass by means of centrifugation and/or filtration approaches;
h) drying the biomass obtained from step g) to obtain a dry mass;
i) weighing the dry mass obtained in step h) and evaluating the chemical properties thereof, preferably by means of approaches for biochemical analysis of pigments, lipids, proteins, and sugars;
j) carrying out a cell culture of microalgae, preferably of the same genus and species used in steps a) - e) using at least one culture medium not previously used, preferably a standard medium, for example BG11, optionally also supplemented with 0.1% acetic acid, and preferably treating the culture in a manner similar to what was described in steps g, h, i);
k) comparing the dry biomass obtained in step i) with the dry biomass obtained in step j) with the following formula:
   weight of dry mass obtained in step i) / weight of dry mass obtained in step j), resulting in a ratio,
wherein the ratio obtained is higher than 2, preferably between 2 and 10.

### EXAMPLE

### Generation of exhausted media

For the present protocol, use was made of exhausted media normally to be discarded, generated by processes tied to the typical activity of a biomedical laboratory. In particular, the waste undergoing investigation was liquid media used for prokaryotic and eukaryotic cell cultures. The waste tested to date derives from:
- Cultures of bacterial cells (*E*. *coli*) for gene amplification or the production of recombinant proteins (hereinafter abbreviated as PCM, prokaryote culture media). The mixture used for these cultures can consist of Luria Bertani (LB) Medium (10 g/L tryptone, 5 g/L yeast extract, 10 g/L NaCl), optionally supplemented with antibiotics (for example ampicillin up to 100 mg/L, kanamycin up to 50 mg/L, chloramphenicol up to 25 mg/L, non-exhaustive list of examples), isopropyl-beta-D-thiogalactopyranoside (up to 1 mg/L), or autoinducing mixtures (10 g/L tryptone, 5 g/L yeast extract, 25 mM Na₂HPO₄, 25 mM KH₂PO₄, 50 mM NH₄Cl, 5 mM Na₂SO₄, 1 mM MgSO₄, 5% glycerol, 0.5% glucose, 2% alpha-lactose), likewise optionally supplemented with the abovementioned antibiotics.
- Cultures of eukaryotic cells (CHO, BHK, HeLa, HEK293, non-exhaustive list of examples) for biomedical research studies or the production of recombinant proteins. In this case the culture media (hereinafter abbreviated as ECM, eukaryote culture media) mainly consist of commercially available formulations. Different formulations based on Dulbecco's Modified Eagle Medium (DMEM) have been tested to date, also with the addition for example of: L-glutamine, glucose, sodium pyruvate, sodium bicarbonate, foetal bovine serum or synthetic analogues (non-exhaustive list of examples). In the case of these culture media as well, antibiotics can also be present (penicillin-streptomycin up to 100 mg/L, G418 up to 500 mg/L, non-exhaustive list of examples).

The media were used by laboratories as per normal cell culture protocols. At the end of their cycle of use, following the removal of cellular material by means of centrifugation and/or filtration processes, instead of being disposed of by means of chemical treatment with hypochlorite or similar disinfectants (e.g. Virkon), they were collected in glass or plastic containers and stored at 4 °C until subsequent treatment.

### Treatment of exhausted media

The exhausted media provided were transferred into Pyrex bottles and subjected to an autoclave sterilisation process at a temperature of 121 °C until a pressure of 2.1 bar was reached for at least 20 minutes. As an alternative to the process of sterilisation by treatment in an autoclave, some exhausted media were subjected to filtration through membranes with a porosity equal to or less than 0.45 µm. Once resterilised, the material was kept closed and stored at 4 and/or 20 °C until its reuse.

### Microalgae cultures

The microalgae used for the example described were the following: *Chlorella vulgaris, Scenedesmus obliquus,* and *Parachlorella kessleri.* Initially, the microalgal strains were cultured in a standard medium (BG11) which was autoclaved (formulation 0A). Composition of BG11: 1500 mg/L NaNO₃, 40 mg/L K₂HPO₄, 75 mg/L MgSO₄, 36 mg/L CaCl₂, 6 mg/L citric acid, 6 mg/L ferric citrate, 1 mg/L EDTA-Na, 20 mg/L Na₂CO₃, 0.061 mg/L H₃BO₃, 0.169 mg/L MnSO₄, 0.287 mg/L ZnSO₄, 0.0025 mg/L CuSO₄, 0.00125 mg/L (NH₄)₆Mo₇O₂₄
BG11 medium supplemented with 0.1% acetic acid (formulation 0B) was used to compare the yields obtained under standard mixotrophic culture conditions.

The microalgae were cultured under sterile conditions inside 100, 250 or 500 mL flasks under stirring (160 RPM) using a stirrer, at a temperature of 26 °C, with a light-dark cycle consisting of 16 hours of light at 100 µmol photons m⁻²s⁻¹ and 8 hours of darkness. Quantification of the microalgae was performed by means of a count with an optical microscope using a Bürker chamber. The initial microalgae concentration for the experiments conducted was always 1 x 10⁶ cell/ml. All inoculation, sampling and dilution operations were performed under a horizontal laminar flow hood to maintain sterility.

The formulations of the exhausted media tested for the growth of the microalgae are shown below.
- Formulation 0A: BG11
- Formulation 0B: BG11 + 0.1% acetic acid
- Formulation 1: ECM-1 100%
- Formulation 2: ECM-1 67% + BG11 33%
- Formulation 3: ECM-1 50% + BG11 50%
- Formulation 4: ECM-1 25% + BG11 75%
- Formulation 5: ECM-1 12.5% + BG11 87.5%
- Formulation 6: PCM-1100%
- Formulation 7: PCM-1 50% + BG11 50%
- Formulation 8: PCM-1 67% + BG11 33%
- Formulation 9: ECM-3 100%
- Formulation 10: ECM-1 50% + PCM-1 50%
- Formulation 11: ECM-1 33% + PCM-1 33% + BG11 33%
- Formulation 12: ECM-1 25% + PCM-1 25% + BG11 50%
- Formulation 13: ECM-2 100%
- Formulation 14: ECM-2 67% + BG11 33%
- Formulation 15: ECM-2 50% + BG11 50%
- Formulation 16: ECM-2 25% + LB 25% + BG11 50%
- Formulation 17: ECM-2 12.5% + LB 12.5% + BG11 75%
- Formulation 18: ECM-3 100%
- Formulation 19: ECM-3 75% + BG11 25%
- Formulation 20: ECM-3 67% + BG11 33%
- Formulation 21: ECM-3 50% + BG11 50%

The experiments were carried out in various steps corresponding to different scale-up levels. A scale-up is a process that allows for gradually increasing volumes from a laboratory scale (from a few ml to hundreds of ml) until arriving at the preparation of cultures in larger volumes (litres or tens of litres).

For an initial evaluation of several formulations simultaneously, use is made of 12-well plates, each well containing 3 mL of a test formulation. After the experiment has been set up under a biological hood, the plates are placed under stirring on a stirrer and the culture conditions used are the same as indicated in the previous paragraph. The algae are allowed to grow for up to 7-10 days.

In this step, monitoring of microalgal growth takes place on a daily basis (1 reading every 24 hours) using a plate reader set to measure the absorption spectrum between 350 and 750 nm. From the absorption spectrum one obtains signals associated with chlorophylls (683 nm) and cellular particulate matter (750 nm). The value recorded was the value resulting from the subtraction of the signal recorded at 683 nm and the one at 750 nm: an increase in this signal, associated solely with the absorption of chlorophylls present within the sample, corresponds to an increase in the microalgal biomass.

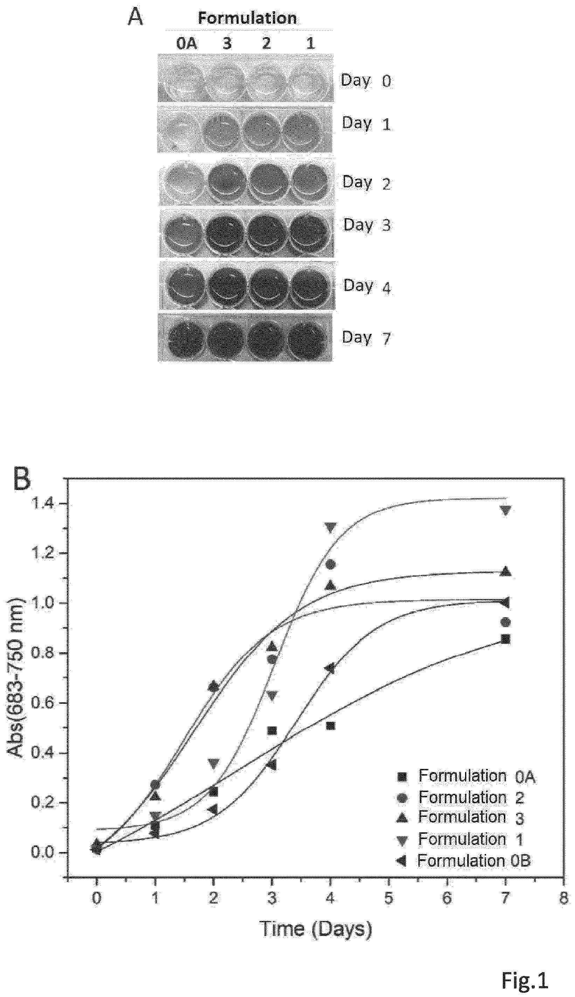

Figure 1 shows an example of a culture of C. *vulgaris* in a 12-well plate containing different formulations. As may be observed from the graph, formulations 2 and 3 provide a significant increase in microalgal growth after 2 days, whilst formulation 1 gives rise to a significant increase in the microalgal concentration compared to the control formulations 0A and 0B after one week. In the former case, it may be inferred that a formulation entirely based on exhausted cell culture media enables the growth of microalgae under conditions that are absolutely competitive compared to standard formulations used for this purpose, and indeed enables a rapid accumulation of biomass if compared to the standard conditions. In the latter case, mixtures supplemented with reformulations of exhausted media show higher yields than those presently obtainable under standard conditions. Absolutely comparable results can be obtained in a reproducible manner using other strains of microalgae.

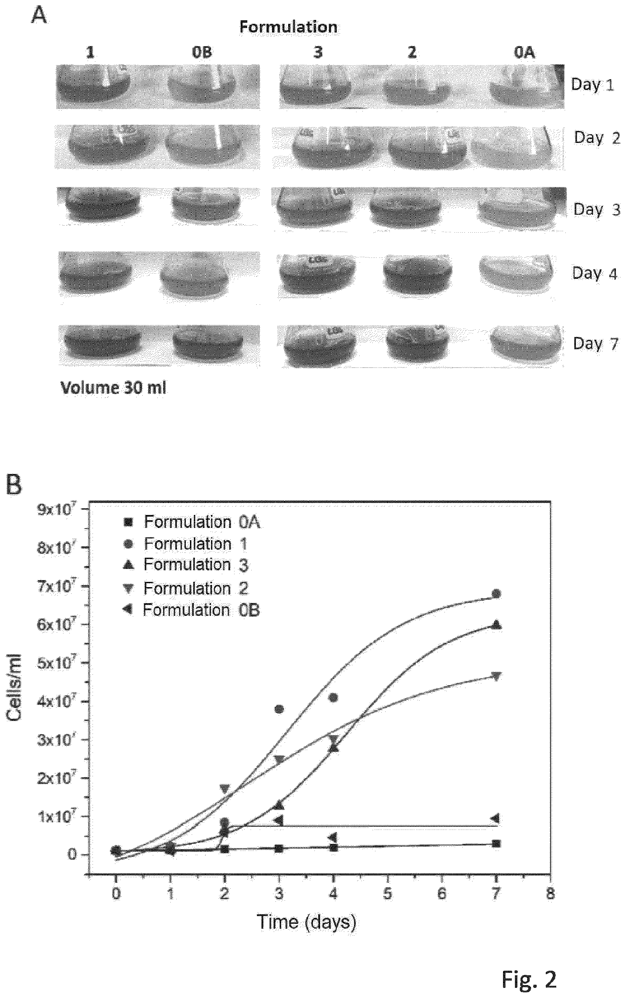

The scale-up of cultures is achieved using glass flasks, for culture volumes of up to 1 L. This enables a more thorough evaluation of the algal biomass over time. Figure 2 shows an example setup of 30 mL cultures in flasks containing different formulations. In this case as well, one observes an increase in growth after 2-3 days in at least two of the tested formulations (1 and 2); moreover, in all three formulations one observes a significant increase in the microalgal concentration compared to the control formulations 0A and 0B after one week.

When the culture volume thus allows, it is possible to draw about 30 mL of culture at regular intervals (after 2, 3, 4 and 7 days, end of the experiment). The biomass was collected by centrifugation (2500 g for 5 min at room temperature). The pellet (which corresponds to the microalgal biomass) is dried in a stove at 65 °C for 2 days and is then weighed to determine its dry weight.

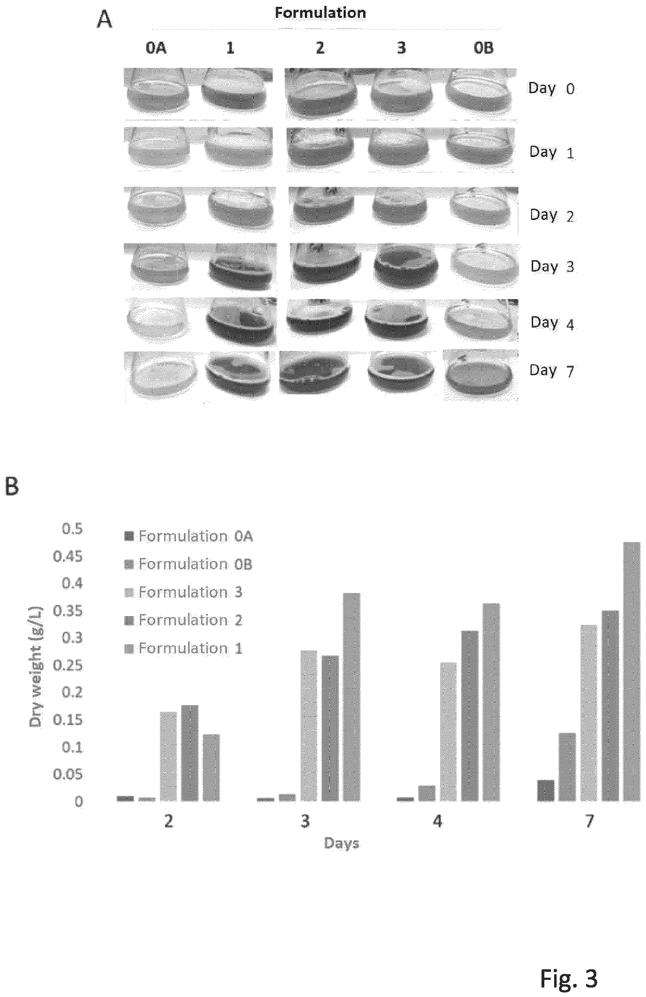

Figure 3 shows an example setup of a 400 mL culture in a flask in which different formulations were analysed. In this case it may clearly be seen that the dry weight of microalgal biomass (g/L) in the formulations we tested increases 2 to 10 times compared to standard conditions depending on the days considered and the formulation used.

The last step for verifying the actual scalability of the microalgae culture process using exhausted culture media provided for 30 L cultures inside a photobioreactor. In this case, the instruments required an adaptation of the culture conditions: temperature: -26° C; light: ~60 µmol photons m⁻² s ⁻¹; cell concentration used: 1x10⁷ cell/ml. An air pump with an output of 30 L/min is connected to the photobioreactor to keep the cells constantly under agitation. Experiments were intentionally conducted also using the photobioreactor under conditions of non-sterility (directly using tap water to prepare the formulations) to verify the actual growth efficiency of the algal biomass under real conditions.

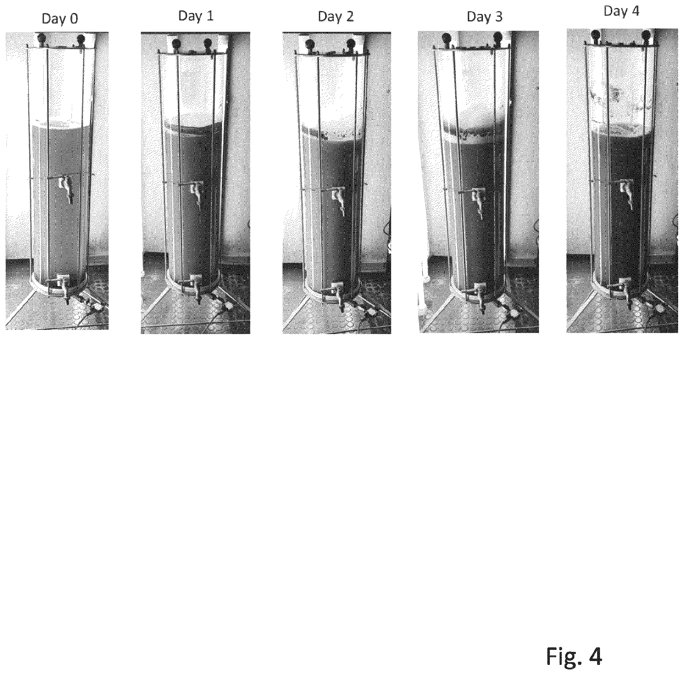

Figure 4 shows the photobioreactor containing 30 litres of microalgal culture in operation.

Moreover, 7 days after the initial inoculation it was decided to proceed with the cultures continuously in the photobioreactor upon collecting about half of the culture and diluting the remainder with an additional 15 litres of formulation containing exhausted media. The yield obtained, calculated in terms of dry biomass (1 ± 0.1 g/L), shows that the formulations based on exhausted media can lend themselves to industrial applications under real conditions, also through continuous processes.

## Claims

1. A process for preparing a cell culture medium comprising the steps of:
(a) providing an exhausted culture medium:
(b) removing cells and/or parts of cells from the exhausted culture medium;
(c) sterilising the exhausted medium obtained in step (b) to obtain a cell culture medium, and
d) optionally mixing the cell culture medium obtained in step c) with at least one nutrient and/or additive;
wherein
sterilisation step c) takes place at a temperature above 100°C for a duration between 5 and 40 minutes and at a pressure between 1 and 3 bar.

2. The process according to claim 1, where sterilisation step c) takes place at a temperature between 100 and 150°C for a duration between 10 and 30 minutes and at a pressure between 1.5 and 3 bar.

3. The process according to claim 1 or 2, wherein sterilisation step c) is performed by filtration with a permeability of less than 0.45 µm, preferably with a permeability of less than 0.2 µm.

4. The process according to any one of claims 1-3, wherein the exhausted culture medium comprises or consists of a prokaryotic cell culture medium optionally supplemented with at least one antibiotic and/or elements suitable for bacterial cell culture.

5. The process according to any one of claims 1-3, wherein the exhausted culture medium comprises or consists of a eukaryotic cell culture medium optionally supplemented with at least one antibiotic and/or elements suitable for eukaryotic cell culture.

6. The process according to any one of claims 1-5, wherein step b) is implemented by means of at least one centrifugation and/or filtration process.

7. A culture medium obtained/obtainable according to any one of claims 1-6.

8. A method for promoting or increasing the growth of an algae cell culture comprising the steps of:
(a) providing an exhausted culture medium;
(b) removing cells and/or parts of cells from the exhausted culture medium;
(c) sterilising the exhausted medium obtained in step (b) to obtain a cell culture medium;
d) optionally mixing the cell culture medium obtained in step c) with at least one nutrient and/or additive;
(e) inoculating a plurality of algae cells into the medium obtained in step (d), and
(f) keeping the plurality of cells in the culture medium for at least 48 hours.

9. The method according to claim 8, wherein the microalgae belong to at least one genus chosen from: Amphora, Anabaena, Anacystis, Apistonema, Arthrospira (Spirulina), Botryococcus, Brachiomonas, Chlamydomonas, Chlorella, Chloroccum, Cruciplacolithus, Cylindrotheca, Coenochloris, Cyanophora, Cyclotella, Dunaliella, Emiliania, Euglena, Extubocellulus, Fragilaria, Galdieria, Goniotrichium, Haematococcus. Halochlorella, Isochyrsis, Leptocylindrus, Micractinium, Melosira, Monodus, Nostoc, Nannochloris, Nannoehloropsis, Navicula, Neospongiococcum, Nitzschia, Odontella, Ochromonas, Ochrosphaera, Pavlova, Parachlorella, Picochlorum, Phaeodactylum, Pleurochyrsis, Porphyridium, Poteriochromonas, Prymnesium. Rhodomonas, Scenedesmus, Skeletonema, Spumella, Stauroneis, Stichococcus, Auxenochlorella, Cheatoceros, Neochloris, Ocromonas, Porphiridium, Synechococcus, Synechocyslis, Tetraselmis, Thraustochytrids, Thalassiosira and species thereof.

10. The method according to claim 9, wherein the microalgae belong to the genus Chlorella, preferably of the species *C*. *vulgaris,* or to the genus Scenedesmus, preferably of the species *S*. *obliquus,* or to the genus Parachlorella, preferably of the species *P. kessleri,* or to the genus Botryococcus, preferably of the species *B. braunii,* and/or to the genus Hematococcus, preferably of the species *H. pluvialis.*

11. The method according to any one of claims 8-10, further comprising the steps of:
(g) collecting the plurality of cells obtained in step (f) preferably by means of centrifugation and/or filtration approaches;
(h) drying the plurality of cells obtained from step (g) to obtain a dry mass,
(i) weighing the dry mass obtained in step (h);
(j) carrying out a cell culture of microalgae, using at least one culture medium not previously used, and
(k) comparing the dry biomass obtained in (i) with the dry biomass obtained in (j) using the following formula:
weight of dry mass obtained in step i) / weight of dry mass obtained in step j), resulting in a ratio,
wherein the ratio obtained is higher than 2, preferably between 2 and 10.
